Europäisches Patentamt

European Patent Office

Office européen des brevets

(11) Veröffentlichungsnummer: **0 248 460**
**B1**

# EUROPÄISCHE PATENTSCHRIFT

(45) Veröffentlichungstag der Patentschrift:
07.02.90

(51) Int. Cl.⁴: **G01N 3/24, G01N 11/10**

(21) Anmeldenummer: 87200838.8

(22) Anmeldetag: 08.05.87

(54) **Kleberprüfgerät.**

(30) Priorität: 03.06.86 DE 8614896 U

(43) Veröffentlichungstag der Anmeldung:
09.12.87 Patentblatt 87/50

(45) Bekanntmachung des Hinweises auf die Patenterteilung:
07.02.90 Patentblatt 90/6

(84) Benannte Vertragsstaaten:
AT BE CH DE ES FR GB GR IT LI LU NL SE

(56) Entgegenhaltungen:
US-A- 4 092 849

(73) Patentinhaber: Brabender oHG, Kulturstrasse 51 - 55,
D-4100 Duisburg(DE)

(72) Erfinder: Sietz, Wolfgang, Dr., Mendelstrasse 10,
D-4100 Duisburg 46(DE)

(74) Vertreter: Berkenfeld, Helmut, Dipl.-Ing., An der
Schanz 2, D-5000 Köln 60(DE)

ACTORUM AG

**Beschreibung**

Die Erfindung betrifft ein Kleberprüfgerät mit zwei übereinander angeordneten und zwischen sich einen Spalt zur Aufnahme einer Probe freilassenden Platten, mit einem Antrieb zum Drehen der einen Platte und mit einer Meßeinrichtung zum Messen der Verdrehung.

Bei der Zubereitung von Teig aus Mehl und Wasser bildet sich eine aus Kleber bestehende elastische Struktur. Bei diesem Kleber handelt es sich um den wasserunlöslichen Eiweißanteil, die Proteinfraktion, des Weizenmehls. Man spricht auch vom Klebereiweiß. Die Menge und die Qualität des Klebers bestimmen im wesentlichen die Eigenschaften des Teiges und die Qualität des aus diesem hergestellten Gebäcks. Die die Qualität des Klebers bestimmenden Eigenschaften sind dessen Dehnbarkeit und Elastizität. Diese Eigenschaften sollen mit dem erfindungsgemäßen Gerät ermittelt werden. Ebenso soll auch Teig geprüft werden können.

Im Stand der Technik hat man die Qualität des Klebers bereits untersucht. In den Labors von Mühlen, Bäckereien, Brotfabriken und dergleichen bestimmt man die sogenannte Feuchtklebermenge. Hierzu wäscht man den Kleber aus dem Teig aus und wiegt ihn. Aus der Menge des Klebers läßt sich jedoch noch nichts über seine Qualität aussagen. In der Weizenstärkeindustrie fällt zusätzlich zu der Stärke auch ein Trockenkleber an. Zur Verbesserung der Teigeigenschaften wird dieser Trockenkleber als Zusatz an Mühlen und Bäckereien verkauft. Auch bei diesem Trockenkleber sind dessen Dehnbarkeit und Elastizität von Interesse.

Zum Prüfen der Qualität des Klebers müssen dessen Dehnungsverhalten und Elastizität ermittelt werden. Eine genaue Ermittlung bedingt eine reproduzierbare Formgebung und Befestigung einer Probe des Klebers. In ihrer Konsistenz gleicht eine Kleberprobe weichem Kaugummi. Infolge dieser Konsistenz hat es im Stand der Technik immer Schwierigkeiten bereitet, einer Kleberprobe eine reproduzierbare Form zu geben und diese reproduzierbar zu befestigen. Versuche, den Kleber in die Form einer Wurst zu rollen und diese an ihren Enden einzuklemmen, führten zu keinem Erfolg.

Bekannt ist ein Kleberprüfgerät (US-A 4 092 849), das zwei zwischen sich einen Ringraum umschließende zylindrische Teile aufweist. Der eine Teil ist mit einem Drehantrieb und der andere Teil mit einer Meßeinrichtung zum Messen einer Verdrehung verbunden. Zum Messen der Schmelz-Elastizität einer Materialprobe wird diese in den Ringraum eingegeben. Das eine Teil wird in Drehung versetzt. über die in den Ringraum eingeschlossene Materialprobe wird diese Drehung auf das andere Teil übertragen. Dieses kann sich über einen begrenzten Winkel mitdrehen. Dann schlägt es an einem Anschlag an. Dadurch wird die Materialprobe geschert. Die dabei auftretenden Scherkräfte werden von der Meßeinrichtung erfaßt. Von diesen kann auf die Schmelz-Elastizität der Materialprobe geschlossen werden. In der US-A 4 092 849 wird erwähnt, daß die Materialprobe auch zwischen zwei Platten eingegeben werden kann.

Von diesem Stand der Technik ausgehend liegt der Erfindung die Aufgabe zugrunde, ein Prüfgerät zu schaffen, mit dem der genannte Kleber, das heißt Feucht- und Trockenkleber, in bezug auf seine Dehnbarkeit und Elastizität reproduzierbar untersucht werden soll. Weiter wird angestrebt, daß sich mit dem Prüfgerät auch Teig prüfen läßt. Zur Lösung dieser Aufgabe sieht die Erfindung bei einem Gerät der eingangs genannten Gattung vor, daß die untere Platte um eine vertikale Achse verdrehbar und die obere Platte gegenüber dieser vertikal verschiebbar und drehfest gehalten ist, die untere Platte an den Antrieb und die Meßeinrichtung angeschlossen und auf der oberen Platte ein bis über den Spalt hinaus verschiebbarer Schneidring geführt ist. Zur Arbeit mit dem Prüfgerät wird eine Probe des Klebers oder des Teiges mit etwas mehr als Sollvolumen auf die untere Platte aufgelegt. Anschließend wird die obere Platte in Richtung auf die untere Platte bewegt. Dabei wird die Probe zusammengedrückt. überschüssiger Kleber oder Teig wird nach außen weggequetscht. Darauf wird der Schneidring nach unten geschoben. Er schneidet den nach außen gequetschten überschüssigen Kleber oder Teig ab. Es ergibt sich eine Probe mit genau vorgegebenem Volumen. Darauf wird der Antrieb eingeschaltet. Die untere Platte verdreht sich gegenüber der oberen Platte. Die zwischen den beiden Platten befindliche Probe verhindert eine kontinuierliche Drehung der unteren Platte. über die Probe klebt sie an der drehfest gehaltenen oberen Platte an. Bei auf die untere Platte konstant einwirkendem Drehmoment bestimmen die Dehnbarkeit und die Elastizität der Probe, wie weit sich die untere Platte im Laufe der Zeit gegenüber der oberen Platte bzw. einer Nullage verdreht. Dabei muß das Drehmoment selbstverständlich so klein gehalten werden, daß es die Dehnbarkeit der Probe nicht übersteigt und die untere Platte durchdreht. Die Meßeinrichtung zeigt die Verdrehung der unteren Platte in Abhängigkeit von der Zeit an. Hieraus lassen sich die Dehnbarkeit und die Elastizität des Klebers oder des Teiges ermitteln.

In einer zweckmäßigen Ausgestaltung ist vorgesehen, daß die untere Platte auf einer vertikal verlaufenden Welle sitzt und diese in einem Lager gehalten ist, der Antrieb und die Meßeinrichtung an die Welle angeschlossen sind und daß die obere Platte an einem Hebel aufgehangen und dieser in einem Lager gelagert ist. Die untere Platte sitzt damit auf dem oberen Ende einer vertikal verlaufenden Welle. Antrieb und Meßeinrichtung können einfach an diese Welle angeschlossen werden. Die obere Platte ist gelenkig an einem verschwenkbaren Hebel befestigt. Damit läßt sie sich einfach durch bloßes Verschwenken des Hebels gegenüber der unteren Platte verschieben. Bei hochgeschwenktem Hebel wird eine Kleberprobe auf die untere Platte aufgelegt oder von dieser abgenommen.

Die untere Platte dreht sich nicht kontinuierlich, sondern wird nur um einen bestimmten Winkel von maximal weniger als etwa 90° gedreht. Ein Elektromotor eignet sich daher nicht als Antrieb. Auch läßt sich das von einem Elektromotor ausgeübte Drehmoment nicht unmittelbar messen. Als Antrieb für die untere Platte ist daher in einer zweckmäßigen Weiterbildung vorgesehen, daß eine Schnurscheibe auf der Welle sitzt, in einem Abstand von dieser eine Umlenkscheibe angeordnet ist, eine Schnur an der Schnurscheibe befestigt, um diese gewunden und um die Umlenkscheibe geführt ist, und ein Gewicht am freien Ende der Schnur befestigt ist. Das an der Schnur hängende und über die Schnurscheibe an der Welle angreifende Gewicht übt ein konstantes Drehmoment aus. Dieses ist der Masse des Gewichtes und dem Durchmesser der Schnurscheibe unmittelbar proportional. Es kann leicht errechnet werden. Mit der Wahl eines größeren oder kleineren Gewichtes läßt sich das Drehmoment auch einfach erhöhen oder absenken. Dem gleichen Zweck dienen an das Gewicht anhängbare Zusatzgewichte.

In einer zweckmäßigen Ausgestaltung besteht das Gewicht aus einem ferromagnetischen Material und liegt im Wirkungsbereich eines Elektromagneten. Dieser kann zum Beispiel eine vertikal verlaufende Bohrung aufweisen. Über dieser ist die Umlenkscheibe angeordnet. Damit wird das Gewicht in der Bohrung geführt. Das Ganze läßt sich dann zum Beispiel so einrichten, daß das Gewicht bei Anlegen einer Spannung an den Elektromagneten in diesem gehalten und die Schnur damit entlastet wird. Durch Abschalten des Elektromagneten wird das Gewicht freigegeben und wirkt auf die Schnurscheibe und damit auf den Kleber ein.

Die Verdrehung der Welle ist ein Maß für die Dehnbarkeit und Elastizität des Klebers. Zur besseren Anzeige dieser Werte ist ein Zeiger an der Welle befestigt. Am freien Ende des Zeigers ist ein Schreibstift angeordnet. Unter diesem läuft ein Meßpapierstreifen ab. Damit wird die Verdrehung über der Zeit aufgetragen.

Damit der Kleber die beiden Platten wirksam zusammenhält und die untere nicht gegenüber der oberen durchrutscht, sind die Oberflächen beider Platten aufgerauht.

Es wurde ausgeführt, daß ein stets gleichbleibendes Volumen des Klebers mit dem Schneidring erreicht wird. Dies ist ein unmittelbar auf dem Umfang der oberen Platte verschiebbarer Ring, dessen Unterkante als Schneide ausgebildet ist. Unter dem oberen Hebel ist zweckmäßig noch ein Anschlag zum Begrenzen der Abwärtsbewegung der oberen Platte vorgesehen. Dieser Anschlag bestimmt den zwischen den beiden Platten offen bleibenden Spalt und damit das Volumen des Klebers.

Am freien Ende des die obere Platte haltenden Hebels befindet sich noch ein Handgriff. Mit diesem kann der Hebel leicht erfaßt und zur Ab- und Aufwärtsbewegung der oberen Platte verschwenkt werden.

Am Beispiel der in der Zeichnung gezeigten Ausführungsform wird die Erfindung nun weiter beschrieben. In der Zeichnung ist:

Fig. 1 eine auseinandergezogene, perspektivische, nicht maßstäbliche Darstellung der wesentlichen Teile des Kleberprüfgerätes,

Fig. 2 eine Aufsicht auf die Oberfläche der unteren Platte in Blickrichtung der Linie II - II in Fig. 1,

Fig. 3 eine vereinfachte schematische Seitenansicht, teilweise im Schnitt, des Gerätes nach dem Einlegen einer Probe zu Beginn eines Arbeitsvorganges,

Fig. 4 eine Seitenansicht ähnlich Fig. 3 nach dem Herunterschwenken des Hebels und der Abwärtsbewegung der oberen Platte,

Fig. 5 eine Seitenansicht ähnlich Fig. 4 nach dem Abschneiden des herausgequetschten Randes der Probe mit dem Schneidring und

Fig. 6 eine Seitenansicht im Betriebszustand des Gerätes.

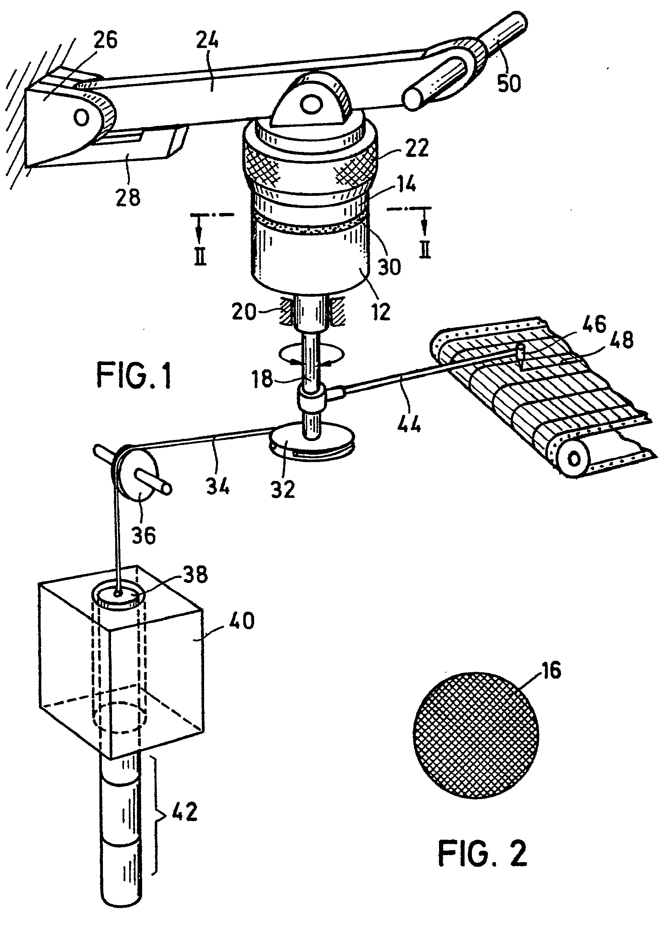

Fig. 1 zeigt das Kleberprüfgerät mit der unteren Platte 12 und der oberen Platte 14. Diese haben die Form von Scheiben oder kurzen Zylindern. Die sich gegenüberliegenden Oberflächen 16 der beiden Platten sind aufgerauht. Fig. 2 zeigt die Oberflä che der unteren Platte 12. Diese sitzt auf einer Welle 18. Diese wird in einem Lager 20 gehalten. Ein Schneidring 22 ist auf dem Umfang der oberen Platte 14 verschiebbar. Die obere Platte 14 ist an dem Hebel 24 aufgehangen. Dieser wird einseitig in einem Lager 26 gehalten. Ein Anschlag 28 hält den Hebel 24 in seiner gezeigten unteren Endstellung. In dieser bleibt zwischen den beiden Platten 12 und 14 ein Spalt offen. In diesem befindet sich die Probe 30. Auf der Welle 18 sitzt eine Schnurscheibe 32. Eine Schnur 34 ist an dieser befestigt und um sie geschlungen. Sie läuft über eine Umlenkscheibe 36. Ein Gewicht 38 hängt am freien Ende der Schnur 34. Das Gewicht 38 wird von einem Elektromagneten 40 umschlossen. Zusatzgewichte 42 lassen sich an das Gewicht 38 anhängen. Ein Zeiger 44 ist an der Welle 18 befestigt. Er trägt einen Schreibstift 46. Unter ihm läuft ein Meßpapierstreifen 48 ab. Ein Handgriff 50 ist noch am freien Ende des Hebels 24 befestigt.

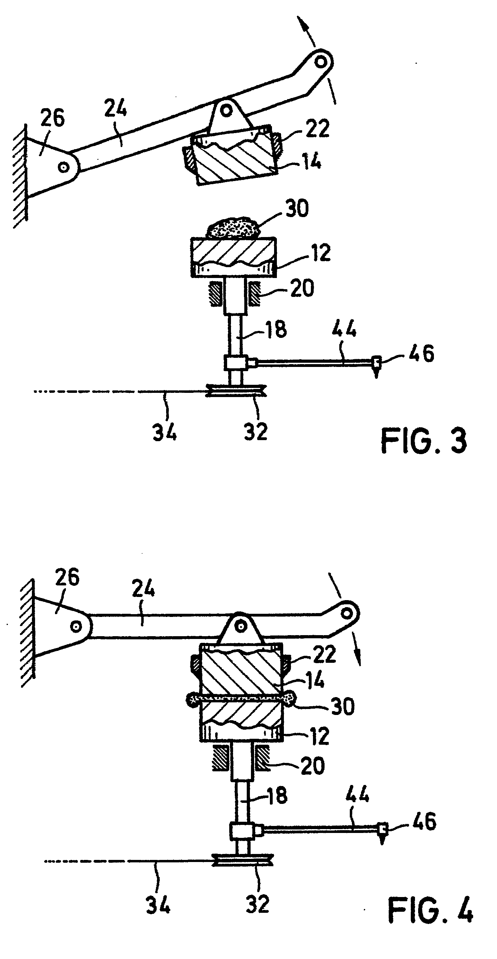

Nach dieser Einzelbeschreibung wird die Arbeit mit dem Gerät unter Bezug auf die Figuren 3 bis 6 erläutert:

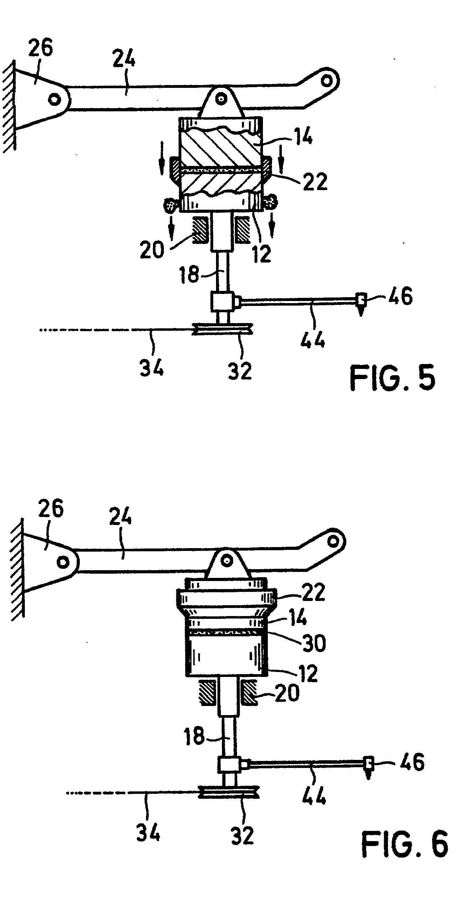

In Fig. 3 befindet sich das Gerät mit hochgeschwenktem Hebel 24 in einer Ruhestellung. Eine Probe 30 wird auf die untere Platte 12 aufgelegt. Die Probe 30 wird zusammengedrückt. Überschüssiger Kleber wird nach außen herausgequetscht. Fig. 4 zeigt dieses Bild. Gemäß der Darstellung in Fig. 5 wird dieser überschüssige Kleber durch Abwärtsbewegung des Schneidringes 22 abgeschnitten und entfernt. Es ergibt sich das in Fig. 6 gezeigte Bild. Die zwischen den beiden Platten 12 und 14 befindliche Probe 30 hat ein vorgegebenes Volumen. Der Durchmesser der scheibenförmigen Probe ergibt sich aus dem Durchmesser der beiden Platten 12 und 14 und des Schneidringes 22. Ihre Höhe ergibt sich aus der Lage des Anschlages 28. Damit ergibt sich das verlangte reproduzierbare Volumen für die Probe 30. Nun wird, was sich wieder mit Blick auf Fig. 1 erkennen läßt, der Elektromagnet 40 betätigt. Das Gewicht 38 kommt, gegebenenfalls mit einem oder mehreren Zusatzgewichten 42, zur Wirkung. Ein Drehmoment greift an der Welle 18 an. Dieses wirkt auch auf die Probe 30 ein. Abhängig von der Dehnbarkeit der Probe 30 wird sich die Welle 18 um einen bestimmten Winkel verdrehen.

Der Schreibstift 46 zeichnet die Verdrehung auf dem Meßpapierstreifen 48 auf.

In Abhängigkeit von den Eigenschaften der Probe zeigen sich bei konstantem Drehmoment bzw. konstanter Scherbelastung unterschiedliche Eigenschaften der Probe. Die Verdrehung wird als Scherwinkel angezeigt und aufgezeichnet. Proben eines gut dehnbaren Klebers werden schnell geschert. Proben eines wenig dehnbaren Klebers werden langsam geschert.

In der eben geschilderten Weise wird die Dehnbarkeit der Probe gemessen. Es wird gemessen und aufgezeichnet, wie stark sich die Probe bei konstantem Drehmoment über der Zeit dehnt. Dies nennt man den Scherwinkel. Ebenso kann auch die Elastizität oder Relaxation gemessen werden. Hierzu werden die Scherbelastung oder das Drehmoment weggenommen. In der Praxis heißt dies, daß der Elektromagnet 40 an Spannung gelegt wird. Er zieht das Gewicht 38 an. Die Schnur 34 wird entlastet. Nun dreht sich die Welle 18 infolge der Elastizität der Probe in umgekehrter Richtung. Im allgemeinen wird die Welle 18 nicht bis in ihre Ausgangsstellung zurückgedreht. Die Zeit, innerhalb der sie sich einer Endstellung praktisch ganz angenähert hat, nennt man die Relaxationszeit. Der Drehwinkel der Welle 18 wird ständig auf dem Meßpapierstreifen 48 aufgezeichnet. Diese Aufzeichnung ermöglicht dann auch das Messen des Winkels zwischen dem ansteigenden und abfallenden Teil des aufgezeichneten Diagramms, das heißt der Drehung in der einen und der Zurückdrehung in der anderen Richtung.

Zum Erleichtern und Automatisieren der Auswertung der Messung können Zeitzähler vorgesehen werden. Diese zeigen die Scherzeit und die Zeit bis zum Erreichen einer vorgegebenen Relaxation unmittelbar nach der Messung automatisch an.

**Patentansprüche**

1. Kleberprüfgerät mit zwei übereinander angeordneten und zwischen sich einen Spalt zur Aufnahme einer Probe (30) freilassenden Platten (12, 14), mit einem Antrieb (32–42) zum Drehen der einen Platte und mit einer Meßrichtung (44, 48) zum Messen der Verdrehung, dadurch gekennzeichnet, daß die untere Platte (12) um eine vertikale Achse verdrehbar und die obere Platte (14) gegenüber dieser vertikal verschiebbar und drehfest gehalten ist, die untere Platte (12) an den Antrieb (32–42) und die Meßeinrichtung (44–48) angeschlossen und auf der oberen Platte (13) ein bis über den Spalt hinaus verschiebbarer Schneidring (22) geführt ist.

2. Kleberprüfgerät nach Anspruch 1, dadurch gekennzeichnet, daß die untere Platte (12) auf einer vertikal verlaufenden Welle (18) sitzt und diese in einem Lager (20) gehalten ist, der Antrieb (32 - 42) und die Meßeinrichtung (44 - 48) an die Welle (18) angeschlossen sind, und daß die obere Platte (14) an einem Hebel (24) aufgehangen und dieser in einem Lager (26) gelagert ist.

3. Kleberprüfgerät nach Anspruch 1 und 2, dadurch gekennzeichnet, daß eine Schnurscheibe (32) auf der Welle (18) sitzt, in einem Abstand von dieser eine Umlenkscheibe (36) angeordnet ist, eine Schnur (34) an der Schnurscheibe (32) befestigt, um diese gewunden und um die Umlenkscheibe (36) geführt ist, und ein Gewicht (38) am freien Ende der Schnur (34) befestigt ist.

4. Kleberprüfgerät nach Anspruch 3, dadurch gekennzeichnet, daß das Gewicht (38) aus einem ferromagnetischen Material besteht und im Wirkungsbereich eines Elektromagneten (40) liegt.

5. Kleberprüfgerät nach Anspruch 3, dadurch gekennzeichnet, daß Zusatzgewichte (42) an das Gewicht (38) anhängbar sind.

6. Kleberprüfgerät nach Anspruch 1 bis 5, dadurch gekennzeichnet, daß ein Zeiger (44) an der Welle (18) befestigt ist.

7. Kleberprüfgerät nach Anspruch 6, dadurch gekennzeichnet, daß am freien Ende des Zeigers (44) ein Schreibstift (46) angeordnet ist und unter diesem ein Meßpapierstreifen (48) abläuft.

8. Kleberprüfgerät nach Anspruch 1 bis 7, dadurch gekennzeichnet, daß die sich gegenüberliegenden Oberflächen (16) der Platten (12, 14) aufgerauht sind.

9. Kleberprüfgerät nach Anspruch 1, dadurch gekennzeichnet, daß der Schneidring (22) ein unmittelbar auf dem Umfang der oberen Platte (14) verschiebbarer Ring mit einer Schneide an seiner Unterkante ist.

10. Kleberprüfgerät nach Anspruch 2, dadurch gekennzeichnet, daß ein Anschlag (28) für den Hebel (24) zum Begrenzen der Abwärtsbewegung der oberen Platte (14) vorgesehen ist.

**Claims**

1. A gluten testing device comprising two plates (12, 14) which are arranged one above the other and which leave a gap free between them for receiving a smple (30), a drive (32–42) for rotating the one plate and a measuring means (44, 48) for measuring the rotary movement characterised in thet the lower plate (12) is rotatable about a vertical axis and the upper plate (14) is held vertically displaceably and non-rotatably relative to the lower plate (12), the lower plate (12) is connected to the drive (32–42) and the measuring means (44–48) and guided on the upper plate (14) is a cutting ring (22) which is displaceable to a position beyond the gap.

2. A gluten testing device according to claim 1 characterised in that the lower plate (12) is carried on a vertically extending shaft (18) and the shaft is held in a bearing (20), the drive (32–42) and the measuring means (44–48) are connected to the shaft (18), and the upper plate (14) is suspended on a lever (24) and the lever is mounted in a mounting (26).

3. A gluten testing device according to claim 1 or claim 2 characterised in that a cord pulley (32) is carried on the shaft (18), a direction-changing pulley (36) is arranged at a spacing from the cord pulley (32), a cord (34) is fixed to the cord pulley (32) and is wound around same and guided around the direction-changing pulley (36), and a weight (38) is fixed to the free end of the cord (34).

4. A gluten testing device according to claim 3 characterised in that the weight (38) comprises a ferromagnetic material and is disposed in the area of action of an electromagnet (40).

5. A gluten testing device according to claim 3 characterised in that additional weights (42) can be hung on the weight (38).

6. A gluten testing device according to one of claims 1 to 5 characterised in that a pointer (44) is fixed to the shaft (18).

7. A gluten testing device according to claim 6 characterised in that a stylus (46) is arranged at the free end of the pointer (44) and a strip of measuring paper (48) passes beneath the stylus.

8. A gluten testing device according to claims 1 to 7 characterised in that the mutually oppositely disposed surfaces (16) of the plates (12, 14) are foughened.

9. A gluten testing device according to claim 1 characterised in that the cutting ring (22) is a ring with a cutting edge at its lower edge, the ring being displaceable directly on the periphery of the upper plate (14).

10. A gluten testing device according to claim 2 characterised in that there is provided an abutment (28) for the lever (24) for limiting the downward movement of the upper plate (14).

**Revendications**

1. Appareil d'essai pour le gluten comportant deux plaques (12, 14) disposées l'une au-dessus de l'autre et ménageant entre elles un espace pouvant recevoir un échantillon (30), un système d'entraînement (32–42) servant à faire tourner l'une des plaques et un dispositif de mesure (44–48) servant à mesurer la rotation, caractérisé en ce que la plaque inférieure (12) peut tourner autour d'un axe vertical et la plaque supérieure (14) peut être déplacée dans le sens vertical par rapport à la plaque inférieure et est calée en rotation, la plaque inférieure (12) étant reliée au système d'entraînement (32–42) et au dispositif de mesure (44–48) et une bague coupante (22) étant guidée sur la plaque supérieure (14) de manière à pouvoir coulisser jusqu'au-delà de l'espace.

2. Appareil d'essai pour le gluten suivant la revendication 1, caractérisé en ce que la plaque inférieure (12) est calée sur un arbre (18) s'étendant verticalement qui est supporté dans un palier (20), le système d'entraînement (32–42) et le dispositif de mesure (44–48) étant reliés à l'arbre (18), et que la plaque supérieure (14) est suspendue à un levier (24) qui est supporté dans un palier (26).

3. Appareil d'essai pour le gluten suivant les revendications 1 et 2, caractérisé en ce que sur l'arbre (18) est calée une poulie (32)) à une certaine distance de laquelle est disposé un disque de renvoi (36), une corde (34) étant fixée à la poulie (32), enroulée autour de celle-ci et guidée autour du disque de renvoi (36), et un poids (38) étant fixé à l'extrémité libre de la corde (34).

4. Appareil d'essai pour le gluten suivant la revendication 3, caractérisé en ce que le poids (38) est constitué d'une matière ferromagnétique et se trouve dans le domaine d'action d'un électroaimant (40).

5. Appareil d'essai pour le gluten suivant la revendication 3, caractérisé en ce que des poids supplémentaires (42) peuvent être suspendus au poids (38).

6. Appareil d'essai pour le gluten suivant les revendications 1 à 5, caractérisé en ce qu'un indicateur (44) est fixé à l'arbre (18).

7. Appareil d'essai pour le gluten suivant la revendication 6, caractérisé en ce qu'à l'extrémité libre de l'indicateur (44) est monté un stylet inscripteur (46) en dessous duquel défile une bande de papier de mesure (48).

8. Appareil d'essai pour le gluten suivant les revendications 1 à 7, caractérisé en ce que les surfaces (16) mutuellement opposées des plaques (12, 14) sont rendues rugueuses.

9. Appareil d'essai pour le gluten suivant la revendication 1, caractérisé en ce que la bague coupante (22) est une bague pouvant être déplacée immédiatement sur la périphérie de la plaque supérieure (14) et présentant un tranchant à son bord inférieur.

10. Appareil d'essai pour le gluten suivant la revendication 2, caractérisé en ce qu'une butée (28) est prévue pour le levier (24) en vue de limiter le déplacement vers le bas de la plaque supérieure (14).

FIG.1

FIG. 2

FIG. 3

FIG. 4

**FIG. 5**

**FIG. 6**